# EUROPEAN PATENT SPECIFICATION

(11) **EP 2 403 335 B1**
(45) Date of publication and mention of the grant of the patent: **20.08.2014**
(21) Application number: 10749258.9
(22) Date of filing: 03.03.2010
(51) Int. Cl.: A61K 45/06, A61K 47/10, A61K 9/00, A61K 9/08, A61K 31/416

(54) **PHARMACEUTICAL COMPOSITION FOR DELIVERY OF RECEPTOR TYROSINE KINASE INHIBITING (RTKI) COMPOUNDS TO THE EYE**
PHARMAZEUTISCHE ZUSAMMENSETZUNG ZUR VERABREICHUNG REZEPTOR-TYROSIN-KINASE-HEMMENDER VERBINDUNGEN INS AUGE
COMPOSITION PHARMACEUTIQUE POUR L'ADMINISTRATION À L' OEIL DE COMPOSÉS INHIBANT LES RÉCEPTEURS TYROSINE KINASE (RTKI)

(30) Priority: 03.03.2009 US 156922 P
(43) Date of publication of application: 11.01.2012
(73) Proprietor: Alcon Research, Ltd., Fort Worth, TX 76134-2099 (US)
(72) Inventor: KABRA, Bhagwati P., Euless, Texas 76039 (US); GHOSH, Malay, Fort Worth, Texas 76109 (US)
(74) Representative: Grünecker, Kinkeldey, Stockmair & Schwanhäusser
(86) International application number: PCT/US2010/026033
(87) International publication number: WO 2010/101992

(56) References cited:
- WO-A1-2007/076358
- WO-A1-2009/014510
- US-A- 5 624 962
- SHOKRI J ET AL: "Improvement of the dissolution rate of indomethacin by a cogrinding technique using polyethylene glycols of various molecular weights", JOURNAL OF DRUG DELIVERY SCIENCE AND TECHNOLOGY 200605 FR, vol. 16, no. 3, May 2006 (2006-05), pages 203-209, XP009160596, ISSN: 1773-2247

## Description

### BACKGROUND OF THE INVENTION

### Field of the Invention

The present invention relates to unique compositions containing compounds with poor solubility and methods useful for treating pathological states that arise or are exacerbated by ocular angiogenesis, inflammation and vascular leakage such as AMD, DR, diabetic macular edema etc., and more specifically, to compositions containing agent with anti-angiogenic, anti-inflammatory or anti-vascular permeability property for use in treating ocular disorders.

### Description of the Related Art

Abnormal neovascularization or angiogenesis and enhanced vascular permeability are major causes for many ocular disorders including age-related macular degeneration (AMD), retinopathy of prematurity (ROP), ischemic retinal vein occlusions, geographic atrophy and diabetic retinopathy (DR). AMD and DR are among the most common cause of severe, irreversible vision loss. In these and related diseases, such as retinal vein occlusion, central vision loss is secondary to angiogenesis, the development of new blood vessels from pre-existing vasculature, and alterations in vascular permeability properties. Geographic atrophy is characterized by patches of atrophy of the retina, retinal pigment epithelium and choroid that are generally round or oval shaped. It is responsible for nearly all cases of severe vision loss associated with nonexudative, or dry, AMD. The patches of atrophy may increase in size and number over time and, in severe cases, coalesce to form larger areas of atrophy.

The angiogenic process is known by the activation of quiescent endothelial cells in pre-existing blood vessels. The normal retinal circulation is resistant to neovascular stimuli, and very little endothelial cell proliferation takes place in the retinal vessels. While there appear to be many stimuli for retinal neovascularization, including tissue hypoxia, inflammatory cell infiltration and penetration barrier breakdown, all increase the local concentration of cytokines (VEGF, PDGF, FGF, TNF, IGF etc.), integrins and proteinases resulting in the formation of new vessels, which then disrupt the organizational structure of the neural retina or break through the inner limiting membranes into the vitreous. Elevated cytokine levels can also disrupt endothelial cell tight junctions, leading to an increase in vascular leakage and retinal edema, and disruption of the organizational structure of the neural retina. Although VEGF is considered to be a major mediator of inflammatory cell infiltration, endothelial cell proliferation and vascular leakage, other growth factors, such as PDGF, FGF, TNF, and IGF etc., are involved in these processes. Therefore, growth factor inhibitors can play a significant role in inhibiting retinal damage and the associated loss of vision upon local delivery in the eye or via oral dosing.

There is no cure for the diseases caused by ocular neovascularization and enhanced vascular permeability. The current treatment procedures of AMD include laser photocoagulation and photodynamic theraphy (PDT). The effects of photocoagulation on ocular neovascularization and increased vascular permeability are achieved only through the thermal destruction of retinal cells. PDT usually requires a slow infusion of the dye, followed by application of non-thermal laser-light. Treatment usually causes the abnormal vessels to temporarily stop or decrease their leaking. PDT treatment may have to be repeated every three months up to 3 to 4 times during the first year. Potential problems associated with PDT treatment include headaches, blurring, and decreased sharpness and gaps in vision and, in 1-4% of patients, a substantial decrease in vision with partial recovery in many patients. Moreover, immediately following PDT treatment, patients must avoid direct sunlight for 5 days to avoid sunburn. Recently, a recombinant humanized IgG monoclonal antibody fragment (ranibizumab) was approved in the US for treatment of patients with age-related macular degeneration. This drug is typically administered via intravitreal injection once a month.

WO-A-2007/076358 relates to an ophthalmic composition for intravitreal injection for treating ocular neovascularization, comprising an active agent in an amount of from about 0.001% to 30% and a polyethylene glycol co-solvent having a molecular weight of from 200 to 2,500.

Many compounds that may be considered potentially useful in treating ocular neovascularization and enhanced vascular permeability-related and other disorders, are poorly soluble in water. A poorly water soluble compound is a substance that is not soluble at a therapeutically effective concentration in an aqueous physiologically acceptable vehicle. Aqueous solubility is an important parameter in formulation development of a poorly water soluble compound. What is needed is a formulation that provides increased solubility of the compound while also providing sufficient bioavailability of the compound so as to maintain its therapeutic potential.

The present invention provides safe and effective formulations for ocular administration of poorly soluble compounds for the treatment of ocular diseases caused by endothelial cell proliferation, vascular leakage, inflammation and angiogenesis.

### SUMMARY OF THE INVENTION

The present invention overcomes these and other drawbacks of the prior art by providing compositions in the form of aqueous solutions for treating ocular diseases due to angiogenesis, enhanced endothelial cell proliferation, inflammation, or increased vascular permeability. Within one aspect of the present invention, a pharmaceutical composition is provided wherein a compound having poor water solubility is incorporated into an aqueous solution containing high concentrations of polyethylene glycol (PEG) having a molecular weight of greater than 2000 for delivery of the compound for use in vitreoretinal therapy, in treating angiogenesis-related ocular disorders, inhibiting neovascularization, controlling vascular permeability, treating inflammation, and improving vision. The aqueous solution of the present invention may be provided to a physician in a pre-filled syringe for administration of the composition to a patient suffering from an angiogenesis-related ocular disorder, neovascularization, vascular permeability, or inflammation.

The bioavailability of the compounds for use in the compositions of the present invention is substantially enhanced via use of a higher molecular weight PEG (e.g., MW ≥ 2000) in the composition. The compositions of the invention are aqueous solutions for delivery through a needle (e.g., 27 gauge) thereby treating angiogenesis-related ocular disorders, inhibiting neovascularization, controlling vascular permeability, treating inflammation, and/or improving vision.

The concentration of the anti-angiogenic, anti-inflammatory, or anti-vascular permeability agent used in the aqueous solutions of the present invention varies depending on the ophthalmic diseases and the route of administration used, and any concentration may be employed as long as its effect is exhibited. Thus, although the concentration is not restricted, a concentration of 0.001% to 10 wt % is preferred. The concentration of PEG will vary depending on the concentration of active used in the formulation. Although the concentrations are not restricted, usually, the preferred concentration of the PEG in the intravitreal composition is from 10% to 55%, more preferred concentration is 15 % to 50 %, and most preferred concentration is 20 % to 50 %.

In another embodiment, posterior juxtascleral (PJ) and periocular (PO) formulations containing (a) an active agent, such as an anti-angiogenic compound, an anti-inflammatory compound, or an anti-vascular permeability agent; (b) a suitable amount of a PEG; (c) a suitable buffer; (d) optionally tonicity agents; and (e) a surfactant are provided. The solutions described herein will preferably be substantially free of ionic species.

In yet another embodiment, the present invention provides formulations for topical ocular dosing, which include (a) a therapeutically effective amount of an active agent, such as an anti-angiogenic agent, an anti-inflammatory compound, or an anti-vascular permeability agent; (b) a surfactant; (c) tonicity agent; (d) PEG; and (e) a buffer.

A wide variety of molecules may be utilized within the scope of present invention, especially those molecules having very low solubility. As used herein, the term "poor solubility" is used to refer to a compound having solubility in water or vehicle that is well below its therapeutic window, typically less than 1000 µg/mL, preferably less than 500 µg/mL, and more preferably less than 200 µg/mL. It is desirable to have a concentration of soluble drug in the formulation such that the concentration of soluble drug in the vitreous is increased. The solutions described herein will preferably contain at least 200 µg/mL, more preferably at least 500 µg/mL, and most preferably at least 1000 µg/mL for local ocular delivery to elicit desirable biological activities.

The compositions of the present invention are preferably administered to the eye of a patient suffering from an angiogenesis or enhanced vascular permeability related ocular, or a disorder characterized by neovascularization or vascular permeability, via posterior juxtascleral administration, intravitreal injection, or vitreoretinal therapy.

### DETAILED DESCRIPTION PREFERRED EMBODIMENTS

As noted above, the present invention provides compositions that contain an active agent having poor water solubility, for use in the treatment of ocular disorders caused by endothelial cell proliferation, enhanced vascular permeability, inflammation, or angiogenesis. The compositions of the invention are useful in treating disorders associated with microvascular pathology, increased vascular permeability and intraocular neovascularization, including diabetic retinopathy (DR), age-related macular degeneration (AMD), geographic atrophy, and retinal edema.

Briefly, within the context of the present invention, an active agent should be understood to be any molecule, either synthetic or naturally occurring, which acts to inhibit vascular growth, reduce vascular permeability, and/or decrease inflammation. In particular, the present invention provides compositions comprising an insoluble, or poorly soluble, active agent in a therapeutically effective amount in an aqueous solution containing high concentrations of high molecular weight PEG (i.e., MW ≥ 2000) for ophthalmic use. As used herein, when referring to a PEG of a particular molecular weight, the term "PEG" will be followed by a number, indicating the molecular weight for that particular PEG. For example, PEG 400 refers to a PEG having a molecular weight of approximately 400. Of course, the skilled artisan will understand that a designation of

PEG 400 will refer to a range of PEGs having molecular weights of about 400 and will encompass PEGs with molecular weights above or below 400 by anywhere from 1-50%

Polyethylene glycols (PEGs) are widely used in a variety of pharmaceutical formulations including parenteral, topical, ophthalmic, oral and rectal preparations. PEGs are stable, hydrophilic substances and are non-irritating to the skin.

The present invention is based, in part, upon the discovery that aqueous solutions incorporating high concentrations of PEGs with higher molecular weights (i.e., MW ≥ 2000) provides a composition that can be delivered directly to the eye of a patient suffering from an ocular disorder via a needle, for example, through a pre-filled syringe.

A higher molecular weight PEG (MW ≥ 2000) is preferred over low molecular weight PEG (e.g., PEG 400) because it keeps tonicity of the formulations within ophthalmically acceptable ranges, even at very high concentrations. This allows for injection of a higher volume of the composition (e.g., 100 µl) into the vitreous of the patient. Higher molecular weight PEGs will also remain in the vitreous for a longer period of time and may provide a higher concentration of the active agent over a longer period of time.

A higher concentration of PEG is preferred because it will increase the solubility of the active agent and will increase the density of the formulations. The density of PEG is about 1.08. Thus, a composition containing a high concentration of a high molecular weight PEG may sink to the bottom of the vitreous when injected into the eye, whereas a composition based on a substance of lower density may remain at the site of injection or float within the vitreous.

An aqueous solution is preferred because it can be filtered to sterilize the composition. Aqueous solutions are also able to be delivered via a needle (e.g., 27 gauge) at room temperature (25 °C). Aqueous solutions will further allow the compositions of the invention to be provided to the ophthalmologist in a pre-filled syringe for ease of delivery to a patient in need thereof.

It is contemplated that any active agent that is poorly water soluble, or any active agent that may benefit from being solubilized within PEG for other reasons, i.e., toxicity, bioavailability, etc., may be included in the compositions of the present invention. For example, anti-angiogenic agents, anti-inflammatory agents, or anti-vascular permeability agents are useful in the compositions of the invention.

Preferred anti-angiogenic agents include, but are not limited to, receptor tyrosine kinase inhibitors (RTKi), in particular, those having a multi-targeted receptor profile such as that described in further detail herein; angiostatic cortisenes; MMP inhibitors; integrin inhibitors; PDGF antagonists; antiproliferatives; HIF-1 inhibitors; fibroblast growth factor inhibitors; epidermal growth factor inhibitors; TIMP inhibitors; insulin-like growth factor inhibitors; TNF inhibitors; antisense oligonucleotides; etc. and prodrugs of any of the aforementioned agents. The preferred anti-angiogenic agent for use in the present invention is a multi-targeted receptor tyrosine kinase inhibitor (RTKi). Most preferred are RTKi's with multi-target binding profiles, such as N-[4-(3-amino-1H-indazol-4-yl) phenyl]-N'-(2-fluoro-5-methylphenyl) urea, having the binding profile substantially similar to that listed in Table 1. Additional multi-targeted receptor tyrosine kinase inhibitors contemplated for use in the compositions of the present invention are described in U.S. Application Serial No. 2004/0235892, incorporated herein by reference. As used herein, the term "multi-targeted receptor tyrosine kinase inhibitor" refers to a compound having a receptor binding profile exhibiting selectivity for multiple receptors shown to be important in angiogenesis, such as the profile shown in Table 1, and described in copending U.S. application serial number 2006/0189608, incorporated herein by reference. More specifically, the preferred binding profile for the multi-targeted receptor tyrosine kinase inhibitor compounds for use in the compositions of the present invention is KDR (VEGFR2), Tie-2 and PDGFR.

**Table 1**

| Kinase Selectivity Profile of a RTK Inhibitor | | | | | | | | | | |
|---|---|---|---|---|---|---|---|---|---|---|
| **KDR** | **FLT1** | **FLT4** | **PDGFR** | **CSF1R** | **KIT** | **FLT3** | **TIE2** | **FGFR** | **EGFR** | **SRC** |
| 4 | 3 | 190 | 66 | 3 | 14 | 4 | 170 | >12,500 | >50,000 | >50,000 |

| | | | | | | | | | | |
|---|---|---|---|---|---|---|---|---|---|---|
| All data reported as IC50 values for kinase inhibition in cell-free enzymatic assays; ND denotes no data. Values determined @ 1 mM ATP. | | | | | | | | | | |

Other agents which will be useful in the compositions and methods of the invention include anti-VEGF antibody (i.e., bevacizumab or ranibizumab); VEGF trap; siRNA molecules, or a mixture thereof, targeting at least two of the tyrosine kinase receptors having IC₅₀ values of less than 200 nM in Table 1; glucocorticoids (i.e., dexamethasone, fluoromethalone, medrysone, betamethasone, triamcinolone, triamcinolone acetonide, prednisone, prednisolone, hydrocortisone, rimexolone, and pharmaceutically acceptable salts thereof, prednicarbate, deflazacort, halomethasone, tixocortol, prednylidene (21-diethylaminoacetate), prednival, paramethasone, methylprednisolone, meprednisone, mazipredone, isoflupredone, halopredone acetate, halcinonide, formocortal, flurandrenolide, fluprednisolone, fluprednidine acetate, fluperolone acetate, fluocortolone, fluocortin butyl, fluocinonide, fluocinolone acetonide, flunisolide, flumethasone, fludrocortisone, fluclorinide, enoxolone, difluprednate, diflucortolone, diflorasone diacetate, desoximetasone (desoxymethasone), desonide, descinolone, cortivazol, corticosterone, cortisone, cloprednol, clocortolone, clobetasone, clobetasol, chloroprednisone, cafestol, budesonide, beclomethasone, amcinonide, allopregnane acetonide, alclometasone, 21-acetoxypregnenolone, tralonide, diflorasone acetate, deacylcortivazol, RU-26988, budesonide, and deacylcortivazol oxetanone); Naphthohydroquinone antibiotics (i.e., Rifamycin); and NSAIDs (i.e., nepafenac, amfenac).

It is contemplated that virtually any PEG with a molecular weight greater than 2000 can be used in the compositions and methods of the invention. Preferred PEGs for use in the compositions and methods of the invention include PEG 4000, PEG 6000, PEG 8000, PEG 14000 and PEG 20000. It is further contemplated that mixtures of higher molecular PEGs may be utilized in the compositions and methods of the invention.

The formulations of the present invention provide a number of advantages over conventional formulations. One advantage of the present invention is that PEGs can successfully solubilize poorly soluble compounds, allowing the preparation of an efficacious ophthalmologically acceptable intravitreal, PJ and/or periocular formulation for local ocular delivery. Additionally, bioavailability of the drug can be modulated by controlling the molecular weight of the PEG used in the formulation. Furthermore, the preparation can be injected using a 27 or 30 gauge needle. Another advantage of the compositions of the present invention is that toxicity of the active compound can be reduced or suitably modulated.

The present inventors have discovered that use of high concentrations of higher molecular weight PEGs to solubilize and deliver highly insoluble anti-angiogenic active compounds provides an efficacious ophthalmic formulation. The use of higher molecular PEGs improves the concentration of the active agent in the solution and improves the bioavailability of the active agent once delivered to a patient. Additionally, the active agent may be delivered to the ocular tissues of a patient treated with the aqueous solutions described herein for a longer period of time than active agents currently used for treatment of such disorders. For example, the aqueous solutions of the present invention are contemplated to deliver active agent to the ocular tissues of a patient for at least two months. In other embodiments of the present invention, the active agent will be delivered to the ocular tissues of the patient for at least three months or for at least four months.

The compound N-[4-(3-amino-1H-indazol-4-yl) phenyl]-N'-(2-fluoro-5-methylphenyl) urea has extremely poor solubility in phosphate buffer, pH 7.2 (0.00059 mg/mL) and would be particularly useful in the solutions of the invention.

In certain preferred embodiments, the formulation of the invention will further comprise a suitable viscosity agent, such as hydroxypropyl methylcellulose, hydroxyethyl cellulose, polyvinylpyrrolilidone, carboxymethyl cellulose, polyvinyl alcohol, sodium chondrointin sulfate, sodium hyaluronate etc. as a dispersant, if necessary. A nonionic surfactant such as polysorbate 80, polysorbate 20, tyloxapol, Cremophor, HCO 40 etc. can be used.

The ophthalmic preparation according to the present invention may contain a suitable buffering system, such as phosphate, citrate, borate, tris, etc., and pH regulators such as sodium hydroxide and hydrochloric acid may also be used in the formulations of the inventions. Sodium chloride or other tonicity agents may be used to adjust tonicity, if necessary. Ophthalmic formulations typically contain buffering agents to maintain pH in a specific range and tonicity agents to impart Osmolality. Buffering agents are generally ionic and may limit the solubilization of an active agent with high molecular weight PEG or promote precipitation of solubilized active agent upon storage. Similarly the most common tonicity agent used in ophthalmic formulations is sodium chloride, which may also limit the solubilization of active agent with high molecular weight PEG. Therefore, in a preferred embodiment the aqueous solutions of the invention are substantially free of ionic species such as buffering agents or tonicity agents. However, optionally they may contain a small amount of acid such as hydrochloride acid or a base such as sodium hydroxide to adjust pH of the active to desired range.

The specific dose level of the active agent for any particular human or animal depends upon a variety of factors, including the activity of the active compound used, the age, body weight, general health, time of administration, route of administration, and the severity of the pathologic condition undergoing therapy.

The formulations described herein may be delivered via intravitreal injection, or via posterior juxtascleral or periocular routes. In preferred embodiments of the present invention, the amount of active agent, or poorly water soluble agent, will be from about 0.001% to 20% for intravitreal administration. More preferably from 0.05% to 5% and most preferably from 0.1% to 3%.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1

### Aqueous Solution Containing High Concentration of PEG 14000

24.5 g PEG 14000 was heated to melting point. 0.5 g of the compound N-[4-(3-amino-1H-indazol-4-yl) phenyl]-N'-(2-fluoro-5-methylphenyl) urea was added to it. The drug completely dissolved in PEG 14000. Hot water was added and stirred. A clear viscous solution was obtained. The warm solution was sterile filtered through a 0.2 micron acrostic syringe filter.

When about 100 µl of this solution is added to water or buffered saline in a 4 ml scintillation vial, it sinks to the bottom and forms a translucent/white mass.

### Examples 2 and 3

The compositions of two non-aqueous solutions of a receptor tyrosine kinase (RTK) inhibitor in low molecular weight PEG are provided in the next Table.

| Examples | 2 | 3 |
|---|---|---|
| Ingredients | W/V% | W/V% |
| RTKi | 3 | 7.5 |
| PEG 400 | 97 | 92.5 |

A pharmacokinetic study was performed in FIX rabbits by giving a 20 µl an injection of non-aqueous PEG based solutions to inferotemporal quadrant of the vitreous. The levels of RTKi observed in the central retina were determined by LC/MS/MS analysis. These levels are provided in the next Table.

| | 2 | 3 |
|---|---|---|
| Examples | | |
| Injection Volume (µl) | 20 | 20 |
| Dose (µg) | 600 | 1500 |
| RTKi concentration (µM) in Retina at Day 2 | 4.6 | 5.0 |
| RTKi concentration (µM) in Retina at Day 14 | 1.7 | 1.5 |
| RTKi concentration (µM) in Retina at Day 56 | 0.34 | 0.86 |

### Examples 4, 5, 6 and 7:

The compositions of a slightly higher molecular weight based PEG solutions are provided in the next Table.

| Examples | 4 | 5 | 6 | 7 |
|---|---|---|---|---|
| Ingredients | W/V% | W/V% | W/V% | W/V% |
| RTKi | 0.6 | 0.3 | 0.6 | 1.2 |
| PEG 400 | 8 | 8 | 8 | 8 |
| Polyethylene Glycol 6000 | - | 21 | 21 | 21 |
| Polyethylene Glycol 20000 | - | 21 | 21 | 21 |
| Polyethylene Glycol 14000 | 41 | - | - | - |
| Water for Injection | Q.s. to 100% | Q.s. to 100% | Q.s. to 100% | Q.s. to 100% |

A pharmacokinetic study was performed in FIX rabbits by giving a 100 µl an injection of the high molecular weight PEG based solutions to inferotemporal quadrant of the vitreous. The levels of RTKi observed in the central retina were determined by LC/MS/MS analysis. These levels are provided in the next Table. The central retina levels from examples 4 to 7 are much higher than those of low molecular PEG based non-aqueous solutions from examples 2 and 3.

| | | | | |
|---|---|---|---|---|
| Injection Volume (µl) | 100 | 100 | 100 | 100 |
| Dose (µg) | 600 | 300 | 600 | 1200 |
| RTKi concentration (µM) in Retina at Day 2 | 46.4 | 7.9 | 13.5 | 25.5 |
| RTKi concentration (µM) in Retina at Day 14 | 19.6 | 3.0 | 4.7 | 9.9 |
| RTKi concentration (µM) in Retina at Day 28 | 16.4 | NT | NT | NT |
| RTKi concentration (µM) in Retina at Day 56 | NT | NT | 15.7 | NT |

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and structurally related may be substituted for the agents described herein to achieve similar results. All such substitutions and modifications apparent to those skilled in the art are deemed to be within the spirit, scope and concept of the invention as defined by the appended claims.

### References

All cited references, to the extent that they provide exemplary procedural or other details supplementary to those set forth herein, are specifically incorporated herein by reference.

## Claims

1. An aqueous solution for treating ocular neovascularization, said composition comprising:
a poorly water soluble active agent in an amount of from 0.01 % to 5%, water and
a polyethylene glycol having a molecular weight of at least 4000 in an amount from 25% to 50%;
wherein the composition is an aqueous composition prepared for intravitreal injection.

2. The aqueous solution of claim 1, wherein the active agent is selected from the group consisting of anti-angiogenic agents, anti-inflammatory agents, and anti-vascular permeability agents.

3. The aqueous solution of claim 2, wherein the active agent is an anti-angiogenic agent.

4. The aqueous solution of claim 3, wherein the anti-angiogenic agent is a multi-targeted receptor tyrosine kinase (RTK) inhibitor.

5. The aqueous solution of claim 4, wherein the RTK inhibitor is N-[4-(3-amino-1H-indazol-4-yl) phenyl]-N'-(2-fluoro-5-methylphenyl) urea.

6. The aqueous solution of claim 5, wherein the said concentration of the anti-angiogenic agent is from 0.1% to 3%.

7. The aqueous solution of claim 6, wherein the PEG is selected from the group consisting of PEG 6000, PEG 20000, and a mixture of PEG 6000 and PEG 20000.

8. The aqueous solution of claim 6, wherein the PEG is selected from the group consisting of PEG 14000 and PEG 20000.

9. The aqueous solution of claim 1, wherein the solution is substantially free of ionic species.

10. The aqueous solution of claim 1, comprising 0.3% (w/v) active agent;
8% (w/v) PEG 400;
21 % (w/v) PEG 6000; and
21% (w/v) PEG 20000;
wherein the solution is substantially free of ionic species.

11. The aqueous solution of claim 1, comprising 0.6% (w/v) active agent;
8% (w/v) PEG 400;
21% (w/v) PEG 6000; and
21% (w/v) PEG 20000;
wherein the solution is substantially free of ionic species.

12. The aqueous solution of claim 1, comprising 1.2% (w/v) active agent;
8% (w/v) PEG 400;
21% (w/v) PEG 6000; and
21% (w/v) PEG 20000;
wherein the solution is substantially free of ionic species.

13. The aqueous solution of claim 1, comprising
0.6% (w/v) active agent; and
41 % (w/v) PEG 14000;
wherein the solution is substantially free of ionic species.

14. The aqueous solution of claim 1, comprising 1% of the active agent N-[4-(3-amino-1H-indazol-4-yl) phenyl]-N'-(2-fluoro-5-methylphenyl) urea and 49% of PEG 14000.

15. The aqueous solution of claim 1 for use in the treatment of an ocular disorder associated with microvascular pathology, increased vascular permeability or intraocular neovascularisation.

16. The aqueous solution of claim 15, wherein said ocular disorder is selected from the group consisting of diabetic retinopathy, age-related macular degeneration, macular edema, uveitis, and geographic atrophy.

17. The aqueous solution of claim 16, wherein the composition is the composition of claim 10.

18. The aqueous solution of claim 16, wherein the composition is the composition of claim 11.

19. The aqueous solution of claim 16, wherein the composition is the composition of claim 12.

20. The aqueous solution of claim 16, wherein the composition is the composition of claim 13.

21. The aqueous solution of claim 16, wherein the composition is the composition of claim 14.

22. The aqueous solution of claim 15, wherein the duration of delivery of the active agent to the ocular tissues of the patient after injection of the solution is at least two months.

## Patentansprüche

1. Eine wässrige Lösung zur Behandlung von Neovaskularisation im Auge, die Zusammensetzung umfassend:
einen schlecht wasserlöslichen Wirkstoff in einer Menge von 0,01% bis 5%, Wasser und
ein Polyethylenglycol mit einem Molekulargewicht von mindestens 4000 in einer Menge von 25% bis 50%;
wobei die Zusammensetzung eine wässrige Zusammensetzung ist, hergestellt für die intravitreale Injektion.

2. Die wässrige Lösung von Anspruch 1, wobei der Wirkstoff ausgewählt ist aus der Gruppe bestehend aus antiantiogenen Mitteln, entzündungshemmenden Mitteln und gefäßdurchlässigkeitshemmenden Mitteln.

3. Die wässrige Lösung von Anspruch 2, wobei der Wirkstoff ein antiantiogenes Mittel ist.

4. Die wässrige Lösung von Anspruch 3, wobei das anitangiogene Mittel ein mehrfachzielgerichteter Inhibitor der Rezeptor-Tyrosinkinase (RTK) ist.

5. Die wässrige Lösung von Anspruch 4, wobei der RTK-Inhibitor N-[4-(3-Amino-1H-indazol-4-yl)phenyl]-N'-(2-fluor-5-methylphenyl)harnstoff ist.

6. Die wässrige Lösung von Anspruch 5, wobei die Konzentration des antiangiogenen Mittels von 0,1% bis 3% beträgt.

7. Die wässrige Lösung von Anspruch 6, wobei das PEG ausgewählt ist aus der Gruppe bestehend aus PEG 6000, PEG 20000 und einer Mischung aus PEG 6000 und PEG 20000.

8. Die wässrige Lösung von Anspruch 6, wobei das PEG ausgewählt ist aus der Gruppe bestehend aus PEG 14000 und PEG 20000.

9. Die wässrige Lösung von Anspruch 1, wobei die Lösung im Wesentlichen frei von ionischen Spezies ist.

10. Die wässrige Lösung von Anspruch 1, umfassend 0,3% (w/v) Wirkstoff;
8% (w/v) PEG 400;
21% (w/v) PEG 6000; und
21% (w/v) PEG 20000;
wobei die Lösung im Wesentlichen frei von ionischen Spezies ist.

11. Die wässrige Lösung von Anspruch 1, umfassend 0,6% (w/v) Wirkstoff;
8% (w/v) PEG 400;
21% (w/v) PEG 6000; und
21% (w/v) PEG 20000;
wobei die Lösung im Wesentlichen frei von ionischen Spezies ist.

12. Die wässrige Lösung von Anspruch 1, umfassend 1,2% (w/v) Wirkstoff;
8% (w/v) PEG 400;
21% (w/v) PEG 6000; und
21% (w/v) PEG 20000;
wobei die Lösung im Wesentlichen frei von ionischen Spezies ist.

13. Die wässrige Lösung von Anspruch 1, umfassend 0,6% (w/v) Wirkstoff; und
41% (w/v) PEG 14000;
wobei die Lösung im Wesentlichen frei von ionischen Spezies ist.

14. Die wässrige Lösung von Anspruch 1, umfassend 1 % des Wirkstoffs N-[4-(3-Amino-1H-indazol-4-yl)phenyl]-N'-(2-fluor-5-methylphenyl)harnstoff und 49% PEG 14000.

15. Die wässrige Lösung von Anspruch 1 zur Verwendung in der Behandlung einer Augenkrankheit, die mit Mikrogefäß-Pathologie, erhöhter Gefäßdurchlässigkeit oder intraokularer Neovaskularisation verbunden ist.

16. Die wässrige Lösung von Anspruch 15, wobei die Augenkrankheit ausgewählt ist aus der Gruppe bestehend aus diabetischer Retinopathie, altersbedingter Makulardegeneration, Makularödem, Uveitis und geographischer Atrophie.

17. Die wässrige Lösung von Anspruch 16, wobei die Zusammensetzung die Zusammensetzung von Anspruch 10 ist.

18. Die wässrige Lösung von Anspruch 16, wobei die Zusammensetzung die Zusammensetzung von Anspruch 11 ist.

19. Die wässrige Lösung von Anspruch 16, wobei die Zusammensetzung die Zusammensetzung von Anspruch 12 ist.

20. Die wässrige Lösung von Anspruch 16, wobei die Zusammensetzung die Zusammensetzung von Anspruch 13 ist.

21. Die wässrige Lösung von Anspruch 16, wobei die Zusammensetzung die Zusammensetzung von Anspruch 14 ist.

22. Die wässrige Lösung von Anspruch 15, wobei die Dauer der Zuführung des Wirkstoffs in die Augengewebe des Patienten nach der Injektion der Lösung mindestens zwei Monate beträgt.

## Revendications

1. Solution aqueuse pour le traitement d'une néovascularisation oculaire, ladite composition comprenant :
un agent actif peu hydrosoluble en une quantité comprise entre 0,01 % et 5 %, de l'eau et
un polyéthylèneglycol ayant une masse moléculaire d'au moins 4000 en une quantité comprise entre 25 % et 50 % ;
laquelle composition est une composition aqueuse préparée pour injection intravitréenne.

2. Solution aqueuse de la revendication 1, dans laquelle l'agent actif est choisi dans le groupe constitué par les agents anti-angiogéniques, les agents anti-inflammatoires, et les agents anti-perméabilité vasculaire.

3. Solution aqueuse de la revendication 2, dans laquelle l'agent actif est un agent anti-angiogénique.

4. Solution aqueuse de la revendication 3, dans laquelle l'agent anti-angiogénique est un inhibiteur multicible des récepteurs à activité tyrosine kinase (RTK).

5. Solution aqueuse de la revendication 4, dans laquelle l'inhibiteur de RTK est la N-[4-(3-amino-1H-indazol-4-yl)phényl]-N'-(2-fluoro-5-méthylphényl)urée.

6. Solution aqueuse de la revendication 5, dans laquelle ladite concentration de l'agent anti-angiogénique est comprise entre 0,1 % et 3 %.

7. Solution aqueuse de la revendication 6, dans laquelle le PEG est choisi dans le groupe constitué par le PEG 6000, le PEG 20 000, et un mélange de PEG 6000 et de PEG 20 000.

8. Solution aqueuse de la revendication 6, dans laquelle le PEG est choisi dans le groupe constitué par le PEG 14 000 et le PEG 20 000.

9. Solution aqueuse de la revendication 1, dans laquelle la solution est pratiquement exempte d'espèces ioniques.

10. Solution aqueuse de la revendication 1, comprenant
0,3 % (p/v) d'agent actif ;
8 % (p/v) de PEG 400 ;
21 % (p/v) de PEG 6000 ; et
21 % (p/v) de PEG 20 000 ;
laquelle solution est pratiquement dépourvue d'espèces ioniques.

11. Solution aqueuse de la revendication 1, comprenant
0,6 % (p/v) d'agent actif ;
8 % (p/v) de PEG 400 ;
21 % (p/v) de PEG 6000 ; et
21 % (p/v) de PEG 20 000 ;
laquelle solution est pratiquement dépourvue d'espèces ioniques.

12. Solution aqueuse de la revendication 1, comprenant
1,2 % (p/v) d'agent actif ;
8 % (p/v) de PEG 400 ;
21 % (p/v) de PEG 6000 ; et
21 % (p/v) de PEG 20 000 ;
laquelle solution est pratiquement dépourvue d'espèces ioniques.

13. Solution aqueuse de la revendication 1, comprenant
0,6 % (p/v) d'agent actif ; et
41 % (p/v) de PEG 14 000 ;
laquelle solution est pratiquement dépourvue d'espèces ioniques.

14. Solution aqueuse de la revendication 1, comprenant 1 % de l'agent actif N-[4-(3-amino-1H-indazol-4-yl)phényl]-N'-(2-fluoro-5-méthylphényl)urée et 49 % de PEG 14 000.

15. Solution aqueuse de la revendication 1 destinée à être utilisée dans le traitement d'un trouble oculaire associé à une pathologie microvasculaire, à une augmentation de la perméabilité vasculaire ou à une néovascularisation intraoculaire.

16. Solution aqueuse de la revendication 15, dans laquelle ledit trouble oculaire est choisi dans le groupe constitué par la rétinopathie diabétique, la dégénérescence maculaire liée à l'âge, l'oedème maculaire, l'uvéite, et l'atrophie géographique.

17. Solution aqueuse de la revendication 16, dans laquelle la composition est la composition de la revendication 10.

18. Solution aqueuse de la revendication 16, dans laquelle la composition est la composition de la revendication 11.

19. Solution aqueuse de la revendication 16, dans laquelle la composition est la composition de la revendication 12.

20. Solution aqueuse de la revendication 16, dans laquelle la composition est la composition de la revendication 13.

21. Solution aqueuse de la revendication 16, dans laquelle la composition est la composition de la revendication 14.

22. Solution aqueuse de la revendication 15, la durée de délivrance de l'agent actif aux tissus oculaires du patient après injection de la solution étant d'au moins deux mois.
